# EUROPEAN PATENT SPECIFICATION

(11) **EP 4 361 621 B1**
(45) Date of publication and mention of the grant of the patent: **08.04.2026**
(21) Application number: 23205990.7
(22) Date of filing: 26.10.2023
(51) Int. Cl.: G01N 27/447

(54) **SAMPLE ANALYSIS METHOD, CAPILLARY ELECTROPHORESIS SOLUTION, AND SAMPLE ANALYSIS KIT**
PROBENANALYSEVERFAHREN, KAPILLARELEKTROFORESELÖSUNG UND PROBENANALYSEKIT
PROCÉDÉ D'ANALYSE D'ÉCHANTILLON, SOLUTION D'ÉLECTROPHORÈSE CAPILLAIRE ET KIT D'ANALYSE D'ÉCHANTILLON

(30) Priority: 28.10.2022 JP 2022173450
(43) Date of publication of application: 01.05.2024
(73) Proprietor: ARKRAY, Inc., Kyoto-shi, Kyoto 601-8045 (JP)
(72) Inventor: OGA, Misaki, Kyoto, 602-0008 (JP)
(74) Representative: Dehns

(56) References cited:
- US-A1- 2010 006 436
- US-A1- 2010 155 242
- US-A1- 2016 209 359

## Description

### BACKGROUND

### Technical Field

The present disclosure relates to a sample analysis method, a capillary electrophoresis solution, and a sample analysis kit.

### Related Art

In the field of clinical examination, proteins such as hemoglobin contained in erythrocytes, albumin, globulin, transferrin or the like contained in serum, and the like are routinely analyzed.

As one of protein analysis methods, a capillary electrophoresis method is used. The sample analysis method using the capillary electrophoresis method is advantageous, for example, in that a small amount of sample is required, and an analyzer can be downsized. In recent years, improvement of analysis accuracy and shortening of analysis time are desired for the above analysis method.

Japanese National-Phase Publication (JP-A) No. 2004-517339 discloses an alkaline pH, free solution capillary electrophoresis method for analyzing a sample containing a protein constituent, the method including at least one step of introducing the sample into a capillary tube including a buffer system, the buffer system further containing at least one additive capable of hydrophobically interacting with one or more protein constituents and capable of providing the protein constituent or constituents with one or more negative charge and of modifying the electrophoretic mobility.

JP-A No. 2005-326407 discloses a free solution capillary electrophoresis method at alkaline pH for analyzing a sample containing protein constituents including one or more lipoprotein constituents, the method including at least one step of introducing a sample into a capillary tube containing an analysis buffer, the analysis buffer further containing at least one anionic surfactant-based additive that hydrophobically interacts with the one or more lipoprotein constituents and modifies the electrophoretic mobility with respect to the mobility of the other protein constituents, a concentration of the additive in the buffer in a range of from 0.001 mM to 0.2 mM.

JP-A No. 2016-136135 and US 2016/209359 A1 disclose a sample analysis method for separating various kinds of hemoglobin in an alkaline solution containing a cationic polymer by capillary electrophoresis.

### SUMMARY

An object of an embodiment of the disclosure is to provide a sample analysis method capable of separating a protein with higher accuracy than in a conventional method, a capillary electrophoresis solution, and a sample analysis kit.

A sample analysis method according to an embodiment of the disclosure includes: a separation step of separating a protein from a sample containing the protein, in an alkaline solution by capillary electrophoresis, in which separation of the protein is performed in the presence of a cationic low-molecular compound having two or more primary amino groups, and the alkaline solution contains a cationic polymer and the cationic compound.

According to an embodiment of the disclosure, it is possible to provide a sample analysis method capable of separating a protein with higher accuracy than in a conventional method, a capillary electrophoresis solution, and a sample analysis kit.

### BRIEF DESCRIPTION OF THE DRAWINGS

Exemplary embodiments of the present disclosure will be described in detail based on the following figures, wherein:
Fig. 1A is a top view illustrating an embodiment of a capillary electrophoresis chip. Fig. 1B is a cross-sectional view of the electrophoresis chip illustrated in Fig. 1A;
Fig. 2 shows an electropherogram obtained in a sample analysis method of Example 1;
Fig. 3 shows an electropherogram obtained in a sample analysis method of Example 2;
Fig. 4 shows an electropherogram obtained in a sample analysis method of Example 3;
Fig. 5 shows an electropherogram obtained in a sample analysis method of Example 4;
Fig. 6 shows an electropherogram obtained in a sample analysis method of Comparative Example 1;
Fig. 7 shows an electropherogram obtained in a sample analysis method of Comparative Example 2;
Fig. 8 shows an electropherogram obtained in a sample analysis method of Comparative Example 3;
Fig. 9 shows an electropherogram obtained in a sample analysis method of Comparative Example 4;
Fig. 10 shows an electropherogram obtained in a sample analysis method of Comparative Example 5;
Fig. 11 shows an electropherogram obtained in a sample analysis method of Comparative Example 6;
Fig. 12 shows an electropherogram obtained in a sample analysis method of Comparative Example 7;
Fig. 13 shows an electropherogram obtained in a sample analysis method of Comparative Example 8;
Fig. 14 shows an electropherogram obtained in a sample analysis method of Comparative Example 9;
Fig. 15 shows an electropherogram obtained in a sample analysis method of Example 5;
Fig. 16 shows an electropherogram obtained in a sample analysis method of Comparative Example 10;
Fig. 17 shows an electropherogram obtained in a sample analysis method of Example 6;
Fig. 18 shows an electropherogram obtained in a sample analysis method of Comparative Example 11;
Fig. 19 shows an electropherogram obtained in a sample analysis method of Example 7;
Fig. 20 shows an electropherogram obtained in a sample analysis method of Example 8; and
Fig. 21 shows an electropherogram obtained in a sample analysis method of Comparative Example 12.

### DETAILED DESCRIPTION

An exemplary embodiment of the present invention will be described. These descriptions and examples illustrate embodiments and do not limit the scope of the invention.

In the present specification, "to" indicating a numerical range is used to mean that numerical values which are described before and after "to" are included as a lower limit value and an upper limit value, respectively.

In a numerical range described in a stepwise manner in the disclosure, an upper limit value or a lower limit value described in one numerical range may be replaced with an upper limit value or a lower limit value described in another numerical range described in a stepwise manner. In a numerical range described in the disclosure, an upper limit value or a lower limit value of the numerical range may be replaced with a value shown in Examples.

Each component may contain a plurality of corresponding substances. When the amount of each component in a composition is referred to, in a case in which a plurality of substances corresponding to each component are present in the composition, the amount of each component in the composition means the total amount of the plurality of substances present in the composition unless otherwise specified.

In the disclosure, the term "step" includes not only an independent step but also a step by which an intended action of the step is achieved, though the step cannot be clearly distinguished from other steps.

In the disclosure, a combination of two or more preferred aspects is a more preferred aspect.

When an embodiment is described with reference to the drawings in the disclosure, the configuration of the embodiment is not limited to the configuration illustrated in the drawings. The sizes of members in each drawing are conceptual, and the relative relationship between the sizes of the members is not limited thereto.

### [Sample analysis method]

A sample analysis method according to an embodiment of the disclosure (hereinafter, also referred to as "specific sample analysis method") includes a separation step of separating a protein from a sample containing the protein, in an alkaline solution by capillary electrophoresis, in which separation of the protein is performed in the presence of a cationic low-molecular compound having two or more primary amino groups (hereinafter, also referred to as "specific low-molecular compound"), and the alkaline solution (hereinafter, also referred to as "specific alkaline solution") contains a cationic polymer.

According to the specific sample analysis method, a protein can be separated with higher accuracy than in a conventional protein separation method. The reason why the above effect is exhibited is not clear, but it is presumed as follows.

Proteins such as hemoglobin, albumin, γ-globulin, and transferrin, which are substances to be analyzed, are negatively charged in an alkaline solution. When a negative electrode is brought into contact with the sample introduction side and a voltage is applied to the substance to be analyzed, these substances to be analyzed move to the positive electrode side due to the charge of the substance to be analyzed and the electroosmotic flow.

On the other hand, since the cationic polymer has a cationic group, the cationic polymer moves from the positive electrode side to the negative electrode side. During electrophoresis, the substance to be analyzed and the cationic polymer repeatedly interact with each other via bonding and dissociation in the capillary, whereby the substance to be analyzed is positively charged, so that a force for pulling back the substance to be analyzed to the negative electrode side is generated. Since the cationic polymer has a large charge, a charge difference between the substances to be analyzed, which have interacted with the cationic polymer, is larger than a charge difference inherent in the substance to be analyzed. Therefore, it is presumed that the substances to be analyzed can be efficiently separated in a short time since a difference in electrophoretic velocity between the substances to be analyzed increases.

The present inventors have found a new problem in that there is variation in the number of cationic polymers that interact with a protein as a substance to be analyzed, and this variation may reduce the separation accuracy.

By separating a protein as a substance to be analyzed in the presence of a specific low-molecular compound, the specific low-molecular compound competes with the cationic polymer and interacts with the substance to be analyzed. As a result, it is presumed that the variation in the number of cationic polymers that interact with a protein as a substance to be analyzed can be reduced, and the separation accuracy is improved.

For the purpose of generating an electroosmotic flow from the negative electrode side toward the positive electrode side in the capillary flow path, the inner wall of the capillary flow path may be coated with a cationic substance. In such a case, the negatively charged substance to be analyzed may be adsorbed to the inner wall. It is presumed that by separating a substance to be analyzed in the presence of a specific low-molecular compound, the adsorption of the substance to be analyzed to the inner wall can be suppressed, and thus the separation accuracy is improved.

### <Separation step>

The specific sample analysis method includes: a separation step of separating a protein from a sample comprising the protein, in a specific alkaline solution by capillary electrophoresis.

The protein is preferably at least one selected from the group consisting of hemoglobin, albumin, γ-globulin, and transferrin.

Separation of a protein from the sample containing the protein, in the specific alkaline solution by capillary electrophoresis can be performed by introducing the sample into a capillary flow path in which the specific alkaline solution is filled, and applying a voltage to the whole or a part of the capillary flow path after introduction of the sample. By applying the voltage, the protein in the sample can be electrophoresed and separated.

The voltage can be applied to the capillary flow path by bringing a negative electrode into contact with the sample introduction side of the capillary flow path and bringing a positive electrode into contact with the specific alkaline solution supply side.

In the specific sample analysis method, separation of the protein is performed in the presence of a specific low-molecular compound.

From the viewpoint of improving the separation accuracy, the specific alkaline solution contains a specific low-molecular compound. The specific low-molecular compound will be described below.

The capillary electrophoresis can be performed by using a device having a capillary flow path.

The cross-sectional shape of the capillary flow path is not particularly limited, and may be a circular shape, a rectangular shape, or other shapes.

In the case of a rectangular shape, each of the flow path height and the flow path width of the capillary flow path is preferably from 1 µm to 1000 µm, more preferably from 10 µm to 200 µm, and further preferably from 25 µm to 100 µm. In the case of a circular shape, the inner diameter of the capillary flow path is preferably 10 µm or more or 25 µm or more, and is preferably 100 µm or less or 75 µm or less.

The flow path length of the capillary flow path is preferably from 10 mm to 150 mm and more preferably from 20 mm to 60 mm.

Examples of the material for the capillary flow path include glass, fused silica, and plastic. Examples of the plastic include polymethyl methacrylate (PMMA), polycarbonate, polystyrene, polytetrafluoroethylene (PTFE), and polyetheretherketone (PEEK).

In the separation step, a capillary electrophoresis chip in which the above-described capillary flow path is made into a microchip may be used.

The capillary electrophoresis chip can include a sample holding tank, an electrophoretic liquid holding tank, and a capillary flow path, in which the sample holding tank and the electrophoretic liquid holding tank are communicated with each other via the capillary flow path.

The size of the capillary electrophoresis chip is not particularly limited, and is preferably appropriately adjusted. The size of the capillary electrophoresis chip can be, for example, from 10 mm to 200 mm in length, from 1 mm to 60 mm in width, and from 0.3 mm to 5 mm in thickness.

The volume of each of the sample holding tank and the electrophoretic liquid holding tank is appropriately determined according to the inner diameter and the length of the capillary flow path, and is preferably from 1 mm³ to 1000 mm³ and more preferably from 5 mm³ to 100 mm³.

The amount of the sample to be filled in the sample holding tank is not particularly limited, and can be from 1 µL to 70 µL.

The amount of the specific alkaline solution to be filled in the electrophoretic liquid holding tank is not particularly limited, and can be from 1 µL to 70 µL.

The voltage applied to both ends of the capillary flow path is preferably from 500 V to 10000 V, and more preferably from 500 V to 5000 V.

The electrophoresis time of capillary electrophoresis in the separation step is preferably from 50 seconds to less than 250 seconds, and more preferably from 60 seconds to 200 seconds.

In the capillary flow path, a liquid flow from the negative electrode side toward the positive electrode side may be generated. Examples of the liquid flow include an electroosmotic flow.

The inner wall of the capillary flow path is preferably coated with a cationic substance or an anionic substance.

By coating the inner wall of the capillary flow path with a cationic substance, the inner wall of the capillary flow path can be positively charged. As a result, an electroosmotic flow from the negative electrode side toward the positive electrode side can be easily generated in the capillary flow path.

When the inner wall of the capillary flow path is coated with the anionic substance, the inner wall of the capillary flow path is negatively charged, but the cationic polymer contained in the specific alkaline solution is bonded to the inner wall of the negatively charged capillary flow path. As a result, the inner wall of the capillary flow path is positively charged, and the electroosmotic flow from the negative electrode side toward the positive electrode side can be easily generated in the capillary flow path as described above.

The cationic substance is not particularly limited, and a silane coupling agent having a cationic functional group and the like can be used. From the viewpoint of improving the separation accuracy, the cationic substance is preferably a polymer having a quaternary ammonium base.

The anionic substance is not particularly limited, and polysaccharides having an anionic group, a silane coupling agent having an anionic functional group, and the like can be used.

Examples of the polysaccharides having an anionic group include sulfated polysaccharides, carboxylated polysaccharides, sulfonated polysaccharides, and phosphorylated polysaccharides.

Examples of the sulfated polysaccharides include chondroitin sulfate, heparin, heparan, fucoidan, and salts thereof. Examples of the carboxylated polysaccharides include alginic acid, hyaluronic acid, and salts thereof.

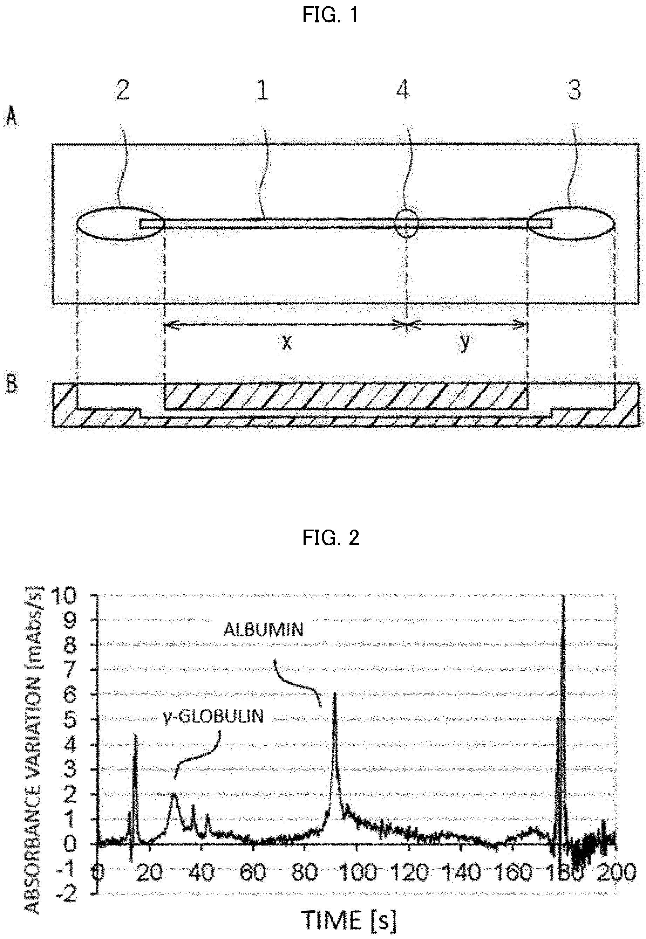

Figs. 1A and 1B illustrate an embodiment of a capillary electrophoresis chip. Fig. 1A is a top view illustrating an embodiment of a capillary electrophoresis chip, and Fig. 1B is a cross-sectional view of the electrophoresis chip illustrated in Fig. 1A.

The capillary electrophoresis chip illustrated in Figs. 1A and 1B includes a capillary flow path 1, a sample holding tank 2, and an electrophoretic liquid holding tank 3, and the sample holding tank 2 and the electrophoretic liquid holding tank 3 are communicated with each other via the capillary flow path 1. The capillary electrophoresis chip has a detection unit 4 for detecting albumin and γ-globulin that have been separated from a sample in the capillary flow path 1. The detection unit 4 may be arranged around a periphery of the capillary flow path 1 (for example, below the capillary flow path 1).

Each of the sample holding tank 2 and the electrophoretic liquid holding tank 3 may includes an electrode for applying a voltage to both ends of the capillary flow path 1 (not illustrated). Specifically, the sample holding tank 2 (sample introduction side) can include a negative electrode, and the electrophoretic liquid holding tank 3 (specific alkaline solution supply side) can include a positive electrode.

The position of the detection unit 4, that is, the length required for separation (distance from the sample holding tank 2 to the detection unit 4, x in Fig. 1A) can be appropriately determined according to the length of the capillary flow path 1 or the like.

When the length (x + y in Fig. 1A) of the capillary flow path 1 is from 10 mm to 150 mm, the distance (x) from the sample holding tank 2 to the detection unit 4 is preferably from 5 mm to 140 mm, more preferably from 10 mm to 100 mm, and further preferably from 15 mm to 50 mm.

### -Specific alkaline solution-

In the disclosure, "alkaline" means that the pH is more than 7.0. The pH of the specific alkaline solution is preferably higher than the isoelectric points of proteins such as hemoglobin, albumin, γ-globulin, and transferrin and is preferably from 8.5 to 12.0, more preferably from 9.0 to 11.0, and further preferably from 9.0 to 10.0.

In the disclosure, the pH of the specific alkaline solution is the pH of the specific alkaline solution at 25°C, and is measured using a pH meter 30 minutes after the electrode is immersed. As the pH meter, F-72 manufactured by HORIBA, Ltd. or a device similar thereto can be used.

### --Cationic polymer--

The specific alkaline solution contains a cationic polymer. The specific alkaline solution may contain two or more cationic polymers.

In the disclosure, the "cationic polymer" means a polymer having a cationic group and a weight average molecular weight of more than 1000.

In the disclosure, the "cationic group" includes a cationic group and a group that is ionized to become a cationic group.

Examples of the cationic group include a primary amino group, a secondary amino group, a tertiary amino group, a quaternary ammonium base, and an imino group. Among them, from the viewpoint of shortening the separation time of proteins, a primary amino group or a secondary amino group is preferable, and a secondary amino group is more preferable.

The cationic polymer is preferably water-soluble. In the disclosure, "water-soluble" means that a target substance is dissolved in an amount of 1 mass% or more in water at 25°C.

Examples of the cationic polymer having primary to tertiary amino groups or a cationic group that can be ionized into primary to tertiary amino groups include polyallylamine, polyvinylamine, polylysine, polyarginine, polyhistidine, polyornithine, polydiallylamine, polymethyldiallylamine, polyethyleneimine, diallylamine-acrylamide polymer, dimethylamine-ammonia-epichlorohydrin polymer, and allylamine-diallylamine polymer.

Examples of the cationic polymer having an imino group or a cationic group that can be ionized into an imino group include polyethyleneimine.

Examples of the cationic polymer having a quaternary ammonium base or a cationic group that can be ionized into a quaternary ammonium base include polyquaternium, dimethylamine-ammonia-epichlorohydrin polymer, dimethylamine-epichlorohydrin polymer, and diallyldimethylammonium chloride polymer.

In the disclosure, "polyquaternium" refers to a cationic polymer including a constituent unit derived from a monomer having a quaternary ammonium group. Polyquaternium can be confirmed by INCI (International Nomenclature for Cosmetic Ingredients) directory. In one or more embodiments, examples of the polyquaternium include polydiallyl dimethyl ammonium salts such as polyquaternium-6 (poly(diallyldimethylammoniumchloride), polyquaternium-7 (copolymer of acrylamide and diallyl dimethyl ammonium chloride), polyquaternium-4 (diallyl dimethyl ammonium chloride-hydroxyethyl cellulose copolymer), and polyquaternium-22 (copolymer of acrylic acid and diallyl dimethyl ammonium chloride), and
diallyldimethylammonium salts such as diallyldimethylammonium chloride; and
polyquaternium-2 (poly[bis(2-chloroethyl)ether-alt-1,3-bis[3-(dimethylamino)propyl]urea]).

As the cationic polymer, in addition to the ammonium salt, in one or more embodiments, a cationic polymer of an onium salt such as a phosphonium salt, an oxonium salt, a sulfonium salt, a fluoronium salt, or a chloronium salt can also be used.

Examples of the cationic polymer having a hydrazide group or a cationic group that can be ionized into a hydrazide group include aminopolyacrylamide.

From the viewpoint of shortening the separation time of proteins, the cationic polymer preferably includes at least one selected from the group consisting of dimethylamine-ammonia-epichlorohydrin polymer, dimethylamine-epichlorohydrin polymer, diallylamine-acrylamide polymer, polyethyleneimine, polyallylamine, diallylamine polymer, allylamine-diallylamine polymer, methyldiallylamine polymer, and diallyldimethylammonium chloride polymer, more preferably includes one or more selected from the group consisting of dimethylamine-ammonia-epichlorohydrin polymer, dimethylamine-epichlorohydrin polymer, diallylamine-acrylamide polymer, polyethyleneimine, and polyallylamine, and further preferably includes one or more selected from the group consisting of dimethylamine-ammonia-epichlorohydrin polymer and diallylamine-acrylamide polymer.

The cationic polymer described above may be in the form of a salt, and examples thereof include hydrochloride.

From the viewpoint of shortening the separation time of proteins, the weight average molecular weight of the cationic polymer is preferably from 10,000 to 500,000, more preferably from 12,000 to 300,000, further preferably from 15,000 to 150,000, particularly preferably from 20,000 to 130,000, and most preferably from 20,000 to 100,000.

The reason why the separation time of proteins is shortened by setting the weight average molecular weight of the cationic polymer within the above numerical range is not clear, but it is presumed as follows.

For example, the isoelectric points of albumin and γ-globulin (IgG as a representative component) are around pH 5 and around pH 5 to 9, respectively.

Since albumin has a lower isoelectric point than γ-globulin, the negative charge of albumin is larger than that of γ-globulin in the alkaline solution, so that the interaction with the cationic polymer is strong.

Therefore, it is presumed that by setting the weight average molecular weight of the cationic polymer to 500,000 or less, it is possible to suppress an excessive increase in difference between the force for pulling back albumin to the negative electrode side and the force for pulling back γ-globulin to the negative electrode side, which are generated by the interaction with the cationic polymer, and it is possible to further shorten the separation time of albumin and γ-globulin.

It is presumed that by setting the weight average molecular weight of the cationic polymer to 10,000 or more, it is possible to suppress an excessive decrease in difference between the force for pulling back albumin to the negative electrode side and the force for pulling back γ-globulin to the negative electrode side, which are generated by the interaction with the cationic polymer, and the separation accuracy can be improved.

In the disclosure, the weight average molecular weight of the cationic polymer refers to the catalogue value. When there is no catalog value of the weight average molecular weight, the weight average molecular weight is a weight average molecular weight in terms of polyethylene glycol measured by a gel permeation chromatography (GPC) method.

From the viewpoint of shortening the separation time of proteins, the content ratio of the cationic polymer with respect to the total mass of the specific alkaline solution is preferably from 0.01 mass% to 10 mass%, more preferably from 0.05 mass% to 8.0 mass%, further preferably from 0.1 mass% to 5.0 mass%, and still more preferably from 1.0 mass% to 3.0 mass%.

As the cationic polymer, one synthesized by a conventionally known method may be used, or a commercially available one may be used.

### --Specific low-molecular compound--

From the viewpoint of improving the separation accuracy of proteins, the specific alkaline solution contains a specific low-molecular compound.

In the disclosure, the "specific low-molecular compound" means a compound having a molecular weight of 1000 or less. When there is a distribution in molecular weight, the molecular weight represents a mass average molecular weight (Mw).

From the viewpoint of improving the separation accuracy of proteins, the molecular weight of the specific low-molecular compound is preferably from 30 to 1000, more preferably from 40 to 800, further preferably from 50 to 400, and still more preferably from 100 to 200.

From the viewpoint of improving the separation accuracy of proteins, the number of primary amino groups of the specific low-molecular compound is preferably from 2 to 5, more preferably from 2 to 4, and further preferably 2 or 3.

From the viewpoint of improving the separation accuracy of proteins, the specific low-molecular compound is at least one selected from the group consisting of 3,3'-diamino-N-methyldipropylamine, N,N'-bis(3-aminopropyl)ethylenediamine, 3,3'-diaminodipropylamine, tris(2-aminoethyl)amine, 1,2-diaminopropane, ethylenediamine, 1,3-diaminopentane, amidol, and 2,2'-oxybis(ethylamine), more preferably at least one selected from the group consisting of 3,3'-diamino-N-methyldipropylamine, N,N'-bis(3-aminopropyl)ethylenediamine, 3,3'-diaminodipropylamine, and tris(2-aminoethyl)amine, and preferably at least one selected from the group consisting of 3,3'-diamino-N-methyldipropylamine and 3,3'-diaminodipropylamine.

From the viewpoint of improving the separation accuracy of proteins, the content ratio of the specific low-molecular compound with respect to the total mass of the specific alkaline solution is preferably from 0.01 mass% to 10 mass%, more preferably from 0.05 mass% to 5 mass%, further preferably from 0.1 mass% to 3 mass%, and still more preferably from 0.3 mass% to 1.0 mass%.

In the specific alkaline solution, the ratio of the content ratio of the specific low-molecular compound to the content ratio of the cationic polymer (the content ratio of the specific low-molecular compound/the content ratio of the cationic polymer) is preferably from 0.001 to 1000, more preferably from 0.01 to 100, further preferably from 0.05 to 10, and still more preferably from 0.1 to 5.

As the specific low-molecular compound, one synthesized by a conventionally known method may be used, or a commercially available one may be used.

### --Water--

The specific alkaline solution may contain water. Examples of water include distilled water, ion-exchanged water, pure water, and ultrapure water.

The content ratio of water with respect to the total mass of the specific alkaline solution is not particularly limited, and can be from 10 mass% to 99.9 mass%.

### --Additive--

The specific alkaline solution may contain additives such as a non-surfactant-type zwitterionic substance (such as non-surfactant-type betaine), a pH buffering substance, and a preservative for suppressing proliferation of microorganisms, and the like.

Examples of the preservative include sodium azide, ethylparaben, and Proclin.

In the disclosure, the "non-surfactant-type zwitterionic substance" means a zwitterionic substance that does not form micelles. In the disclosure, "not forming micelles" means not forming micelles in an aqueous medium or not substantially forming micelles. In the disclosure, "not substantially forming micelles" means that the critical micelle concentration is preferably 200 mmol/L or more and more preferably 300 mmol/L or more, and further preferably, a zwitterionic substance has not critical micelle concentration.

In the disclosure, "zwitterionic substance" means a compound having a positively charged group and a negatively charged group at positions not adjacent to each other in the same molecule, having no hydrogen atom capable of being dissociated bonded to an atom having a positive charge, and having no charge as a whole molecule.

### -Sample-

The sample contains a protein.

The protein is preferably one or more selected from the group consisting of hemoglobin, albumin, γ-globulin, and transferrin.

Examples of hemoglobin include HbA0, HbA1c, HbA2, HbE, HbG, HbA, HbS, HbF, HbC, and HbD.

Examples of γ-globulin include IgG, IgM, IgA, IgD, and IgE.

The content of the protein with respect to the total mass of the sample is not particularly limited, and can be set to from 0.001 mass% to 100 mass%.

The form of the sample is not particularly limited, and may be those prepared from a sample raw material or may be a sample raw material itself.

Examples of the sample raw material include raw materials containing proteins, and biological samples.

Examples of the biological sample include blood, serum, plasma, or those prepared from these, and blood derivatives containing erythrocyte components and the like.

Examples of blood, serum, and plasma include blood, serum, and plasma collected from a living body, and include blood, serum, and plasma of mammals other than human, and blood, serum, and plasma of human.

The blood derivatives containing erythrocyte components are separated or prepared from blood and examples thereof include those containing erythrocyte components. Examples thereof include a blood cell fraction from which plasma is removed, a blood cell concentrate, a lyophilizate of blood or blood cells, a hemolysis sample obtained by hemolysis of whole blood, centrifuged blood, naturally precipitated blood, and washed blood cells.

The sample may contain the specific low-molecular compound, but it is preferable that the sample does not contain the specific low-molecular compound from the viewpoint of the separation accuracy and the like.

From the viewpoint of improving the separation accuracy of proteins, the sample preferably contains an alkaline solution containing a cationic polymer.

The sample containing the alkaline solution can be obtained by diluting the sample raw material using the alkaline solution. The dilution rate is preferably from 1.2 times to 100 times, more preferably from 2 times to 60 times, and further preferably from 3 times to 50 times on a volume basis. The material used for dilution is not particularly limited, and examples thereof include a pH adjusting agent (for example, hydrochloric acid or the like), a surfactant (for example, EMULGEN LS-110 (manufactured by Kao Corporation) or the like), an antiseptic (for example, sodium azide or the like), an ionic strength adjusting agent (for example, sodium chloride or the like), and a refractive index adjusting agent (for example, saccharides such as sucrose).

The alkaline solution may be the same as or different from the specific alkaline solution filled in the capillary flow path.

The sample may contain a non-surfactant-type sulfobetaine.

In the disclosure, the "non-surfactant-type sulfobetaine" means sulfobetaine that does not form micelles.

In the disclosure, "not forming micelles" means not forming micelles in an aqueous medium or not substantially forming micelles. In the disclosure, "not substantially forming micelles" means that the critical micelle concentration is preferably 200 mmol/L or more and more preferably 300 mmol/L or more, and further preferably, a zwitterionic substance has not critical micelle concentration.

In the disclosure, "betaine" means a compound having a positively charged group and a negatively charged group at positions not adjacent to each other in the same molecule, having no hydrogen atom capable of being dissociated bonded to an atom having a positive charge, and having no charge as a whole molecule.

Examples of the positively charged group of the non-surfactant-type sulfobetaine include a quaternary ammonium cation group, a sulfonium group, and a phosphonium group. Among them, from the viewpoint of shortening the separation time of albumin and γ-globulin and improving the separation accuracy, a quaternary ammonium cation group is preferable.

The number of carbon atoms present between the positively charged group and the negatively charged group is preferably from 1 to 10, more preferably from 2 to 8, and further preferably from 2 to 5.

From the viewpoint of shortening the separation time of albumin and γ-globulin and improving the separation accuracy, the molecular weight of the non-surfactant-type sulfobetaine is preferably from 100 to 500, more preferably from 120 to 400, and further preferably from 170 to 300.

Examples of the non-surfactant-type sulfobetaine include 3-(1-pyridinio)propanesulfonate (the first one from top in left in the chemical formulae as set forth below), dimethyl ethyl ammonium propane sulfonate (the second one from top in left in the chemical formulae as set forth below), 3-[(2-hydroxyethyl)dimethylammonio]propane-1-sulfonate (the third one from top in left in the chemical formulae as set forth below), 3-(4-tert-butyl-1-pyridinio)propanesulfonate (the fourth one from top in left in the chemical formulae as set forth below), N-methyl-N-(3-sulfopropyl)morpholinium (the fifth one from top in left in the chemical formulae as set forth below), dimethylbenzylammonium propane sulfonate (the first one from top in right in the chemical formulae as set forth below), and 3-(1-methylpiperidinio)-1-propanesulfonate (the second one from top in right in the chemical formulae as set forth below). The non-surfactant-type sulfobetaine is not limited to these compounds.

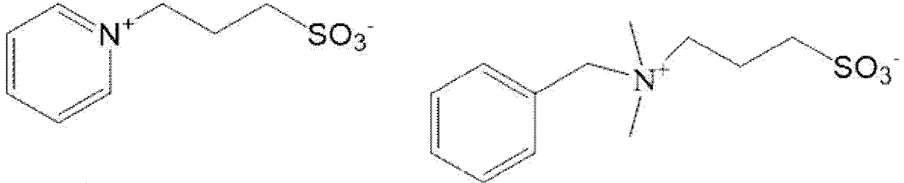

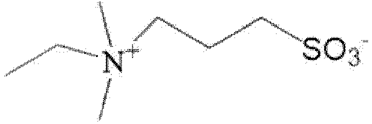

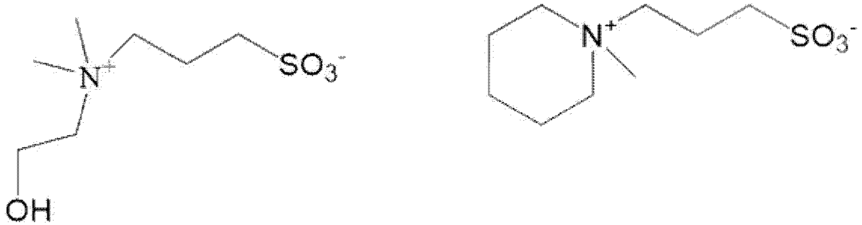

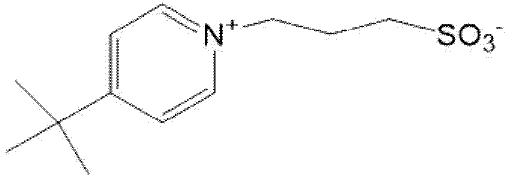

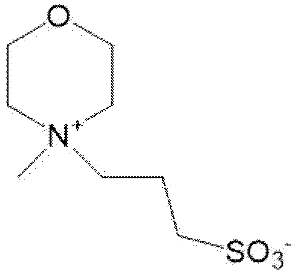

Examples of commercially available products of the compounds exemplified above include NDSB-201 (3-(1-pyridinio)propanesulfonate), NDSB-195 (dimethylethylammonium propane sulfonate), NDSB-211 (3-[(2-hydroxyethyl)dimethylammonio]propane-1-sulfonate), NDSB-256 (dimethylbenzylammonium propane sulfonate), NDSB-221 (3-(1-methylpiperidinio)-1-propanesulfonate, NDSB-256-4T (3-(4-tert-butyl-1-pyridinio)propanesulfonate, and NDSB-223 (N-methyl-N-(3-sulfopropyl)morpholinium) manufactured by Merck Corporation.

Among the compounds exemplified above, from the viewpoint of shortening the separation time of albumin and γ-globulin and
improving the separation accuracy, the following compounds are preferable.

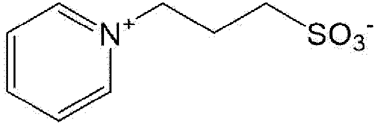

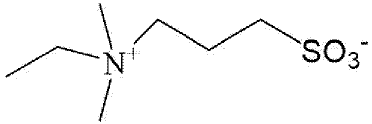

As the non-surfactant-type sulfobetaine, one synthesized by a conventionally known method may be used, or a commercially available one may be used.

From the viewpoint of shortening the separation time of albumin and γ-globulin and improving the separation accuracy, the content ratio of the non-surfactant-type sulfobetaine with respect to the total mass of the sample is preferably from 0.1 mass% to 30 mass%, more preferably from 0.5 mass% to 20 mass%, further preferably from 1 mass% to 15 mass%, and still more preferably from 1 mass% to 9 mass%.

### <Detection step>

The specific sample analysis method can include a detection step of detecting the protein separated in the separation step.

The protein can be detected by an optical method. Examples of the detection by an optical method include measurement of absorbance.

More specifically, the protein can be detected by irradiating the separated protein with light having a wavelength of 280 nm to obtain an absorbance spectrum in which the vertical axis represents absorbance and the horizontal axis represents time.

When a capillary electrophoresis chip is used for separating a protein, it is preferable to irradiate the detection unit with light having a wavelength of 280 nm.

A protein may be detected using an electropherogram (differential waveform) obtained by differentiating the waveform of the absorbance spectrum with respect to time.

The fact that the specific sample analysis method is excellent in separation accuracy as compared with the conventional method can be confirmed by the value of the maximum absorbance variation at the peak of protein, whether the peak in the electropherogram is broad, and the like.

An embodiment of the specific sample analysis method will be described with reference to Figs. 1A and 1B. The specific sample analysis method is not limited to that described below.

First, the specific alkaline solution containing a cationic polymer is filled as an electrophoretic liquid in the electrophoretic liquid holding tank 3 of the capillary electrophoresis chip, and the specific alkaline solution is filled in the capillary flow path 1 by capillary action.

Next, the sample is added to the sample holding tank 2 of the capillary electrophoresis chip in which the specific alkaline solution is filled.

The sample to be added to the sample holding tank 2 can be prepared by diluting a biological sample, which is a sample raw material, with the alkaline solution.

A negative electrode is brought into contact with the sample holding tank 2 and a positive electrode is brought into contact with the electrophoretic liquid holding tank 3 (not illustrated), and a voltage is applied to both ends of the capillary flow path 1, that is, between the sample holding tank 2 and the electrophoretic liquid holding tank 3. As a result, the sample is introduced from the sample holding tank 2 into the capillary flow path 1, the sample containing a protein moves from the sample holding tank 2 toward the electrophoretic liquid holding tank 3, and the protein is separated.

In the detection unit 4, a protein is detected by irradiation with light having a wavelength of 280 nm and measurement of absorbance by an absorbance measuring device.

The sample analysis method of the disclosure can be used for prevention, diagnosis, treatment, and the like of multiple myeloma, nephrotic syndrome, liver cirrhosis, nutritional disorder, abnormal Hb disease, β-thalassemia, and the like.

### [Capillary electrophoresis solution]

A capillary electrophoresis solution according to the invention contains a cationic polymer and a specific low-molecular compound and is used for separation of a protein by capillary electrophoresis.

A preferred embodiment of the capillary electrophoresis solution is the same as the specific alkaline solution used in the specific sample analysis method, and thus the description thereof is omitted here.

### [Sample analysis kit]

A sample analysis kit according to the invention includes: a container containing the above-described capillary electrophoresis solution; and an electrophoresis chip including a sample holding tank, an electrophoretic liquid holding tank, and a capillary flow path, in which the sample holding tank and the electrophoretic liquid holding tank are communicated with each other via the capillary flow path.

A preferred embodiment of the electrophoresis chip is the same as that of the electrophoresis chip used in the specific sample analysis method, and thus the description thereof is omitted here.

Examples of the material for the container containing the capillary electrophoresis solution include glass, fused silica, and plastic. The plastic has been described above, and thus the description thereof is omitted here.

### EXAMPLES

Examples will be described below, but the disclosure is not limited to these Examples at all. In the following description, unless otherwise specified, "part(s)" and "%" are all on a mass basis.

In the following Examples and Comparative Examples, electrophoresis was performed by a continuous sample introduction method.

### <Separation device and measuring apparatus>

As a separation device, a resin chip having the capillary flow path 1 with the structure illustrated in Fig. 1 (flow path width: 40 µm, flow path height: 40 µm, flow path length: 30 mm, distance (x) from the sample holding tank 2 to the detection unit 4: 20 mm) was used. The capacity of each of the sample holding tank 2 and the electrophoretic liquid holding tank 3 was set to 60 µL. The inner wall of the capillary flow path was coated with polydiallyldimethylammonium chloride.

As a measuring device, an electrophoresis device self-manufactured was used.

### <<Example 1>>

The following substances were mixed, and 3-hydroxypropanesulfonic acid and water were added until the pH reached 9.8 to prepare a specific alkaline solution (capillary electrophoresis solution) 1. The content ratio of the cationic polymer with respect to the total mass of the specific alkaline solution 1 was set to 1.5 mass%, and the content ratio of the specific low-molecular compound was set to 0.58 mass%.

### (Composition of specific alkaline solution 1)

· Dimethylamine-ammonia-epichlorohydrin polymer 1 (cationic polymer, KHE102L manufactured by SENKA corporation, weight average molecular weight: from 20,000 to 100,000) 1.5 mass%
· Specific low-molecular compound 1 (3,3'-diamino-N-methyldipropylamine (hereinafter, also referred to as "DAMDPA" and represented by the following chemical formula), molecular weight: 145.25) 0.58 mass%
· Sodium azide 0.02 mass%
· EMULGEN LS-110 (manufactured by Kao Corporation) 0.1 mass%
· 3-Hydroxypropanesulfonic acid
· Water

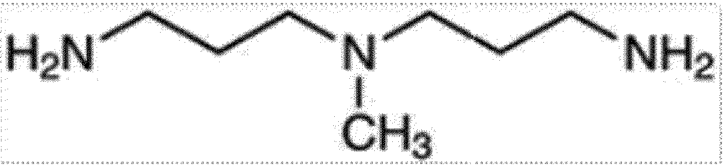

A human biological serum sample containing albumin and γ-globulin was prepared, and diluted (dilution rate 7 times (volume basis)) with a diluent 1 (pH 8.8) having the following composition to obtain Sample A.

In the diluent 1, hydrochloric acid and water were added until the pH of the solution reached 8.8.

### (Diluent 1)

· Polyethyleneimine (cationic polymer, manufactured by FUJIFILM Wako Pure Chemical Corporation, weight average molecular weight: 70,000) 0.75 mass%
· Sucrose 0.787 mass%
· Sodium chloride 0.26 mass%
· Sodium azide 0.018 mass%
· EMULGEN LS-110 (manufactured by Kao Corporation) 0.4 mass%
· Hydrochloric acid
· Water

To the electrophoretic liquid holding tank 3, 60 µL of the specific alkaline solution 1 was added, and the specific alkaline solution 1 was filled in the capillary flow path 1 by capillary action.

To the sample holding tank 2, 60 µL of Sample A was added.

Next, a negative electrode was brought into contact with the sample holding tank 2, a positive electrode was brought into contact with the electrophoretic liquid holding tank 3, and electrophoresis was started by applying a voltage under constant current control of 75 µA.

During electrophoresis, the detection unit 4 was irradiated with light of 280 nm, and the absorbance was measured to obtain an absorbance spectrum. An electropherogram was obtained by differentiating the waveform of the absorbance spectrum with respect to time. Electrophoresis was performed for 200 seconds. The obtained electropherogram is shown in Fig. 2.

A prototype self-manufactured was used for light irradiation, absorbance measurement, and electropherogram acquisition.

In Table 1, the maximum absorbance variation for each of albumin and γ-globulin was summarized.

### <<Example 2>>

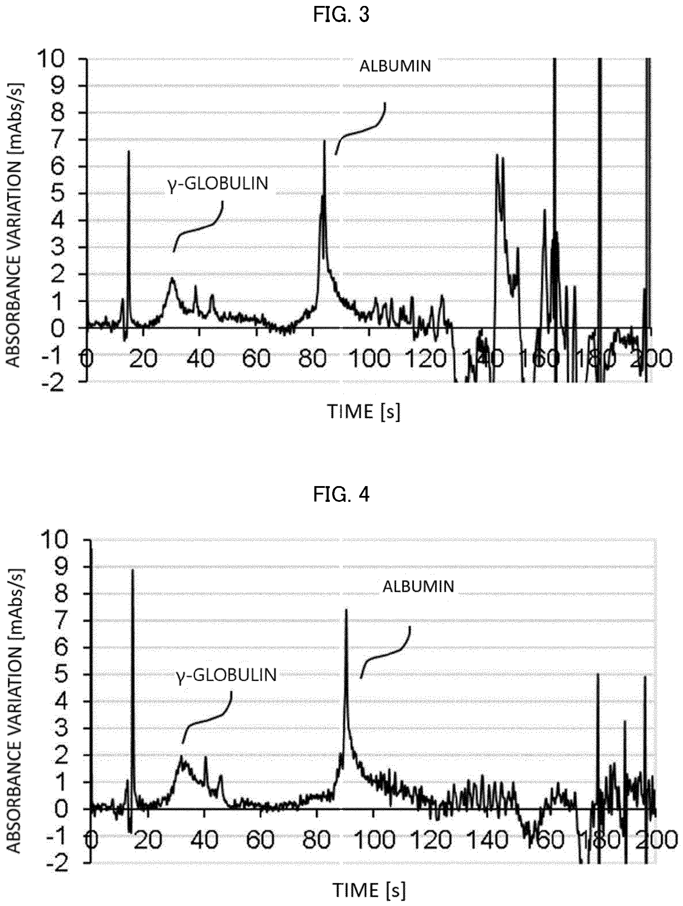

An electropherogram was obtained in the same manner as in Example 1, except that the specific low-molecular compound 1 was changed to a specific low-molecular compound 2 represented by the following chemical formula (N,N'-bis(3-aminopropyl)ethylenediamine (hereinafter, also referred to as "BAPEDA"), molecular weight: 174.29), and the content ratio was changed to 0.70 mass%. The obtained electropherogram is shown in Fig. 3.

In Table 1, the maximum absorbance variation for each of albumin and γ-globulin was summarized.

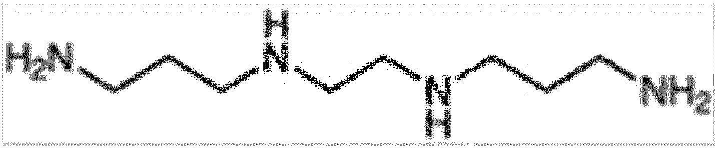

### <<Example 3>>

An electropherogram was obtained in the same manner as in Example 1, except that the specific low-molecular compound 1 was changed to a specific low-molecular compound 3 represented by the following chemical formula (3,3'-diaminodipropylamine (hereinafter, also referred to as "DADPA"), molecular weight: 131.22), and the content ratio was changed to 0.52 mass%. The obtained electropherogram is shown in Fig. 4.

In Table 1, the maximum absorbance variation for each of albumin and γ-globulin was summarized.

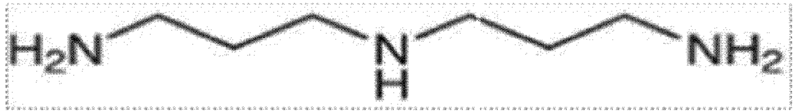

### <<Example 4>>

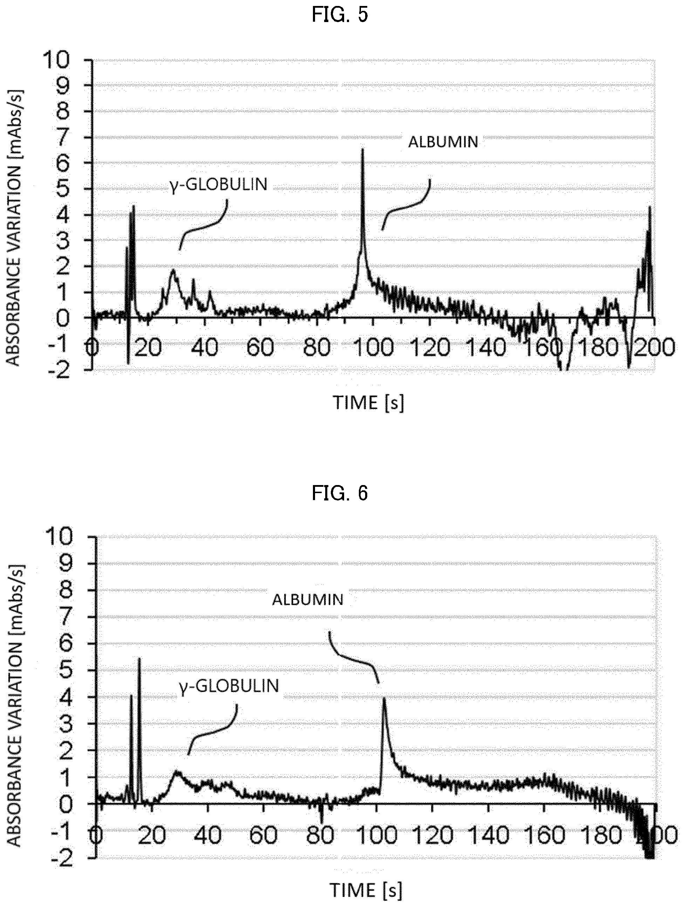

An electropherogram was obtained in the same manner as in Example 1, except that the specific low-molecular compound 1 was changed to a specific low-molecular compound 4 (tris(2-aminoethyl)amine (hereinafter, also referred to as "TAA"), molecular weight: 146.24), and the content ratio was changed to 0.58 mass%. The obtained electropherogram is shown in Fig. 5.

In Table 1, the maximum absorbance variation for each of albumin and γ-globulin was summarized.

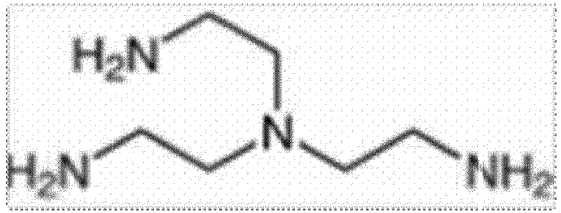

### <<Comparative Example 1>>

An electropherogram was obtained in the same manner as in Example 1, except that the specific alkaline solution 1 was changed to an alkaline solution 10 having the following composition. The obtained electropherogram is shown in Fig. 6.

In the alkaline solution 10, sodium hydroxide and water were added until the pH of the solution reached 9.8.

In Table 1, the maximum absorbance variation for each of albumin and γ-globulin was summarized.

### (Composition of alkaline solution 10)

· Dimethylamine-ammonia-epichlorohydrin polymer 1 (cationic polymer, KHE102L manufactured by SENKA corporation, weight average molecular weight: from 20,000 to 100,000) 1.5 mass%
· Sodium azide 0.02 mass%
· EMULGEN LS-110 (manufactured by Kao Corporation) 0.1 mass%
· Sodium hydroxide
· Water

### <<Comparative Example 2>>

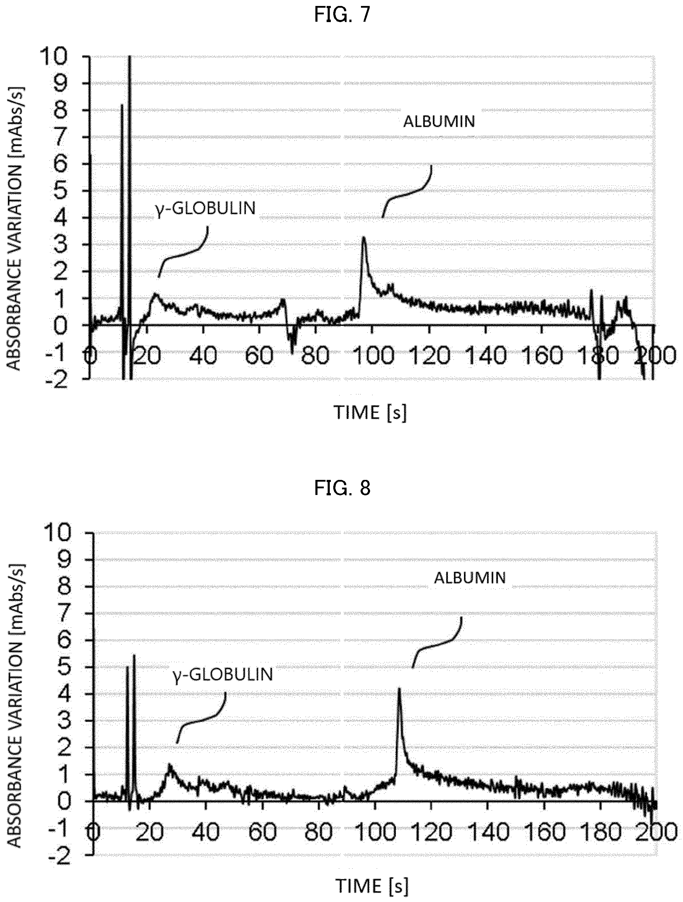

An electropherogram was obtained in the same manner as in Example 1, except that the specific alkaline solution 1 was changed to an alkaline solution 11 having the following composition. The obtained electropherogram is shown in Fig. 7.

In the alkaline solution 11, sodium hydroxide and water were added until the pH of the solution reached 9.8.

In Table 1, the maximum absorbance variation for each of albumin and γ-globulin was summarized.

The content ratio of the cationic polymer with respect to the total mass of the alkaline solution 11 was set to 1.5 mass%, and the content ratio of the specific low-molecular compound was set to 0.69 mass%.

### (Composition of alkaline solution 11)

· Dimethylamine-ammonia-epichlorohydrin polymer 1 (cationic polymer, KHE102L manufactured by SENKA corporation, weight average molecular weight: from 20,000 to 100,000) 1.5 mass%
· Low-molecular compound 11 (N,N,N',N",N"-pentamethyldiethylenetriamine (hereinafter, also referred to as "PMDETA" and represented by the following chemical formula), molecular weight: 173.30) 0.69 mass%
· Sodium azide 0.02 mass%
· EMULGEN LS-110 (manufactured by Kao Corporation) 0.1 mass%
· Sodium hydroxide
· Water

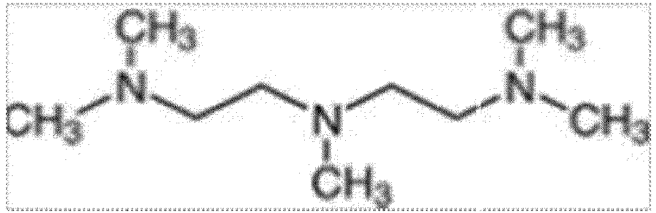

### <<Comparative Example 3>>

An electropherogram was obtained in the same manner as in Example 1, except that the specific low-molecular compound 1 was changed to a low-molecular compound 12 represented by the following chemical formula (diethylamine (hereinafter, also referred to as "DEA"), molecular weight: 73.14), and the content ratio was changed to 0.29 mass%. The obtained electropherogram is shown in Fig. 8.

In Table 1, the maximum absorbance variation for each of albumin and γ-globulin was summarized.

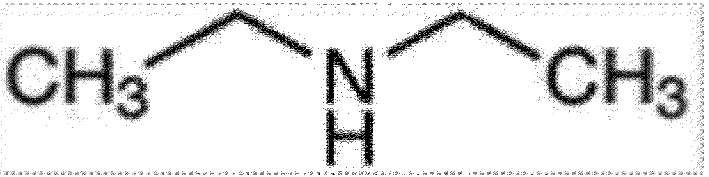

### <<Comparative Example 4>>

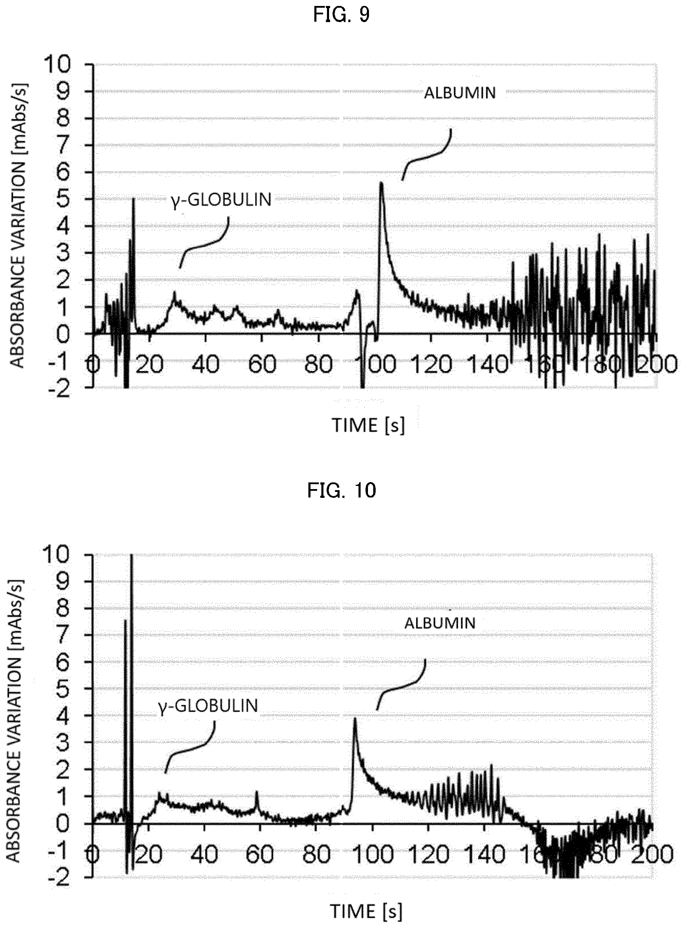

An electropherogram was obtained in the same manner as in Example 1, except that the specific low-molecular compound 1 was changed to a low-molecular compound 13 represented by the following chemical formula (tetraethylammonium hydroxide (hereinafter, also referred to as "TEA"), molecular weight: 147.26), and the content ratio was changed to 0.59 mass%. The obtained electropherogram is shown in Fig. 9.

In Table 1, the maximum absorbance variation for each of albumin and γ-globulin was summarized.

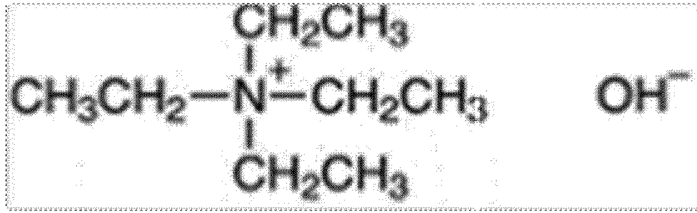

### <<Comparative Example 5>>

An electropherogram was obtained in the same manner as in Example 1, except that the low-molecular compound 11 was changed to a low-molecular compound 14 represented by the following chemical formula (diethanolamine (hereinafter, also referred to as "DEtOHA"), molecular weight: 105.14), and the content ratio was changed to 0.42 mass%. The obtained electropherogram is shown in Fig. 10.

In Table 1, the maximum absorbance variation for each of albumin and γ-globulin was summarized.

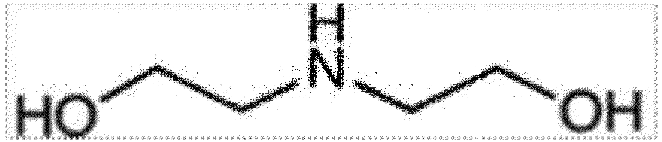

### <<Comparative Example 6>>

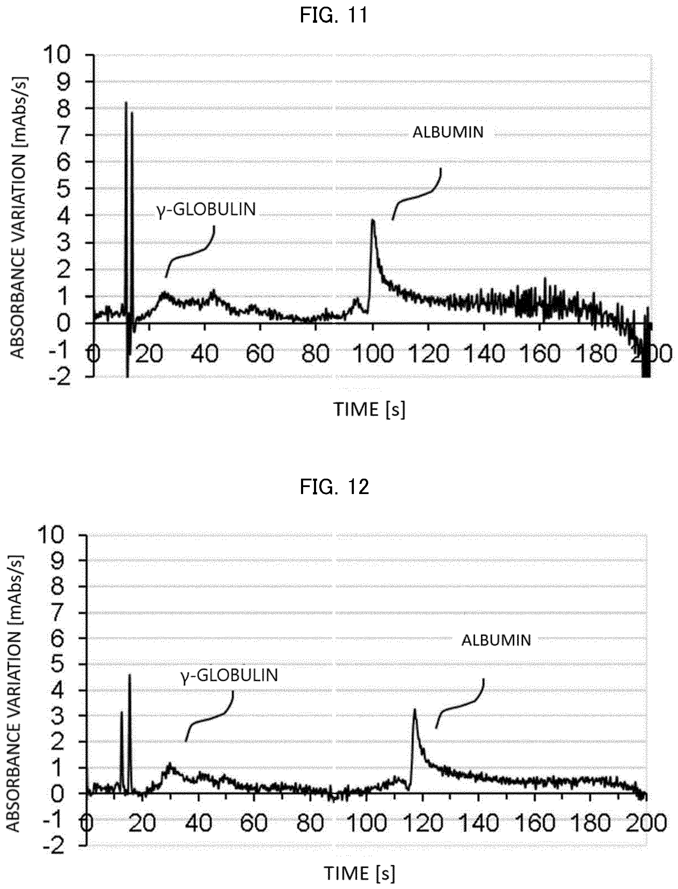

An electropherogram was obtained in the same manner as in Comparative Example 2, except that the low-molecular compound 12 was changed to a low-molecular compound 15 represented by the following chemical formula (N-methyldiethanolamine (hereinafter, also referred to as "MDEA"), molecular weight: 119.16), and the content ratio was changed to 0.48 mass%. The obtained electropherogram is shown in Fig. 11.

In Table 1, the maximum absorbance variation for each of albumin and γ-globulin was summarized.

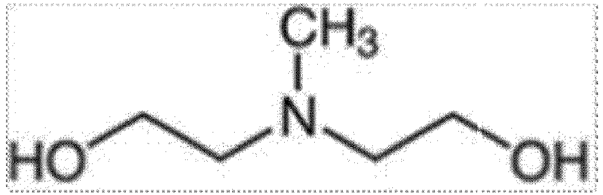

### <<Comparative Example 7>>

An electropherogram was obtained in the same manner as in Comparative Example 2, except that the low-molecular compound 12 was changed to a low-molecular compound 16 represented by the following chemical formula (2-amino-2-hydroxymethyl-1,3-propanediol (hereinafter, also referred to as "TRIS"), molecular weight: 121.14), and the content ratio was changed to 0.48 mass%. The obtained electropherogram is shown in Fig. 12.

In Table 1, the maximum absorbance variation for each of albumin and γ-globulin was summarized.

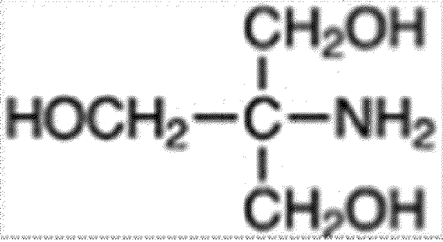

### <<Comparative Example 8>>

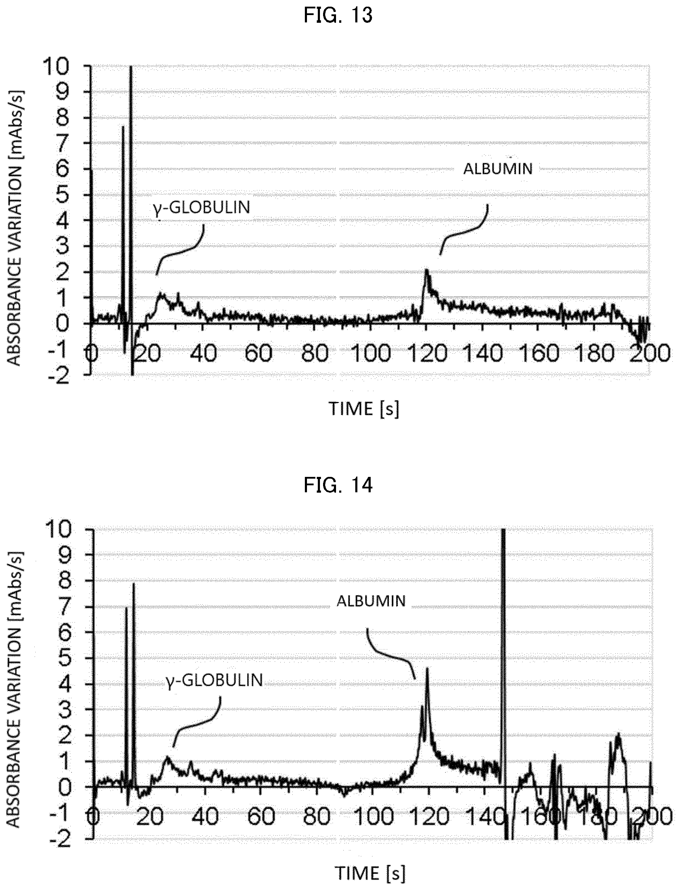

An electropherogram was obtained in the same manner as in Comparative Example 2, except that the low-molecular compound 12 was changed to a low-molecular compound 17 represented by the following chemical formula (N-(2-hydroxypropyl)ethylenediamine (hereinafter, also referred to as "HPEDA"), molecular weight: 118.18), and the content ratio was changed to 0.47 mass%. The obtained electropherogram is shown in Fig. 13.

In Table 1, the maximum absorbance variation for each of albumin and γ-globulin was summarized.

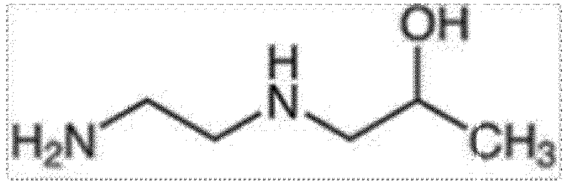

### <<Comparative Example 9>>

An electropherogram was obtained in the same manner as in Example 1, except that the specific low-molecular compound 1 was changed to a low-molecular compound 18 represented by the following chemical formula (N,N'-dimethylethylenediamine (hereinafter, also referred to as "DMEDA"), molecular weight: 88.15), and the content ratio was changed to 0.35 mass%. The obtained electropherogram is shown in Fig. 14.

In Table 1, the maximum absorbance variation for each of albumin and γ-globulin was summarized.

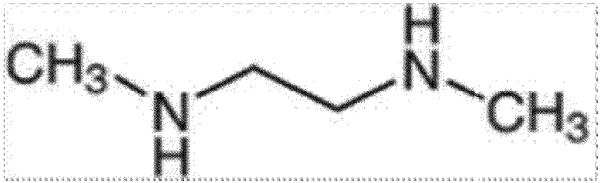

**[Table 1]**

| | Low-molecular compound | | Maximum absorbance variation (mAbs/s) | |
|---|---|---|---|---|
| | Type | Number of primary amino groups | Albumin | γ-Globulin |
| Example 1 | DAMDPA | 2 | 6.09 | 2.01 |
| Example 2 | BAPEDA | 2 | 6.96 | 1.87 |
| Example 3 | DADPA | 2 | 7.40 | 1.97 |
| Example 4 | TAA | 3 | 6.55 | 1.88 |
| Comparative Example 1 | - | - | 3.98 | 1.27 |
| Comparative Example 2 | PMDETA | 0 | 3.28 | 1.19 |
| Comparative Example 3 | DEA | 0 | 4.21 | 1.41 |
| Comparative Example 4 | TEA | 0 | 5.63 | 1.57 |
| Comparative Example 5 | DEtOHA | 0 | 3.92 | 1.17 |
| Comparative Example 6 | MDEA | 0 | 3.86 | 1.17 |
| Comparative Example 7 | TRIS | 1 | 3.26 | 1.19 |
| Comparative Example 8 | HPEDA | 1 | 2.09 | 1.19 |
| Comparative Example 9 | DMEDA | 0 | 4.61 | 1.18 |

From Figs. 2 to 14 and Table 1, it is found that in the sample analysis method of Examples in which the separation of albumin and γ-globulin was performed using an alkaline solution containing a cationic polymer and a cationic low-molecular compound having two or more primary amino groups, as compared with the sample analysis method of Comparative Examples in which the separation of albumin and γ-globulin was performed using an alkaline solution not containing a cationic low-molecular compound having two or more primary amino groups, the maximum absorbance variation of each of albumin and γ-globulin was large, each peak in the drawing was not broad, and thus the separation accuracy was excellent.

### <<Example 5>>

A sample containing hemoglobin was prepared, and diluted (dilution rate 7 times (volume basis)) with the diluent 1 (pH 8.8) to obtain Sample C.

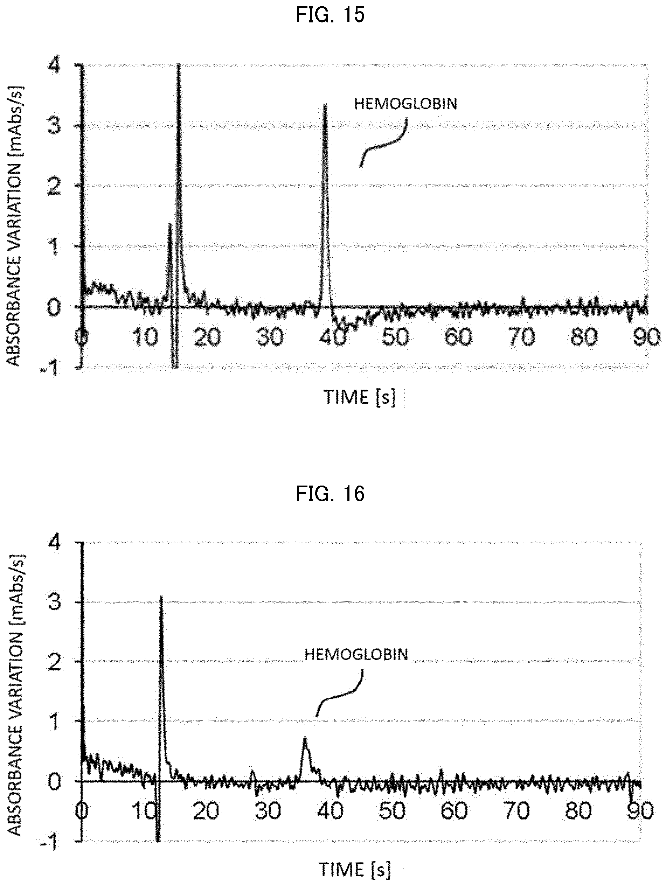

An electropherogram was obtained in the same manner as in Example 1, except that Sample A was changed to Sample C. The obtained electropherogram is shown in Fig. 15.

In Table 2, the maximum absorbance variation for hemoglobin was summarized.

### <<Comparative Example 10>>

An electropherogram was obtained in the same manner as in Example 1, except that Sample A was changed to Sample C. The obtained electropherogram is shown in Fig. 16.

In Table 2, the maximum absorbance variation for hemoglobin was summarized.

**[Table 2]**

| | Low-molecular compound | | Maximum absorbance variation (mAbs/s) |
|---|---|---|---|
| | Type | Number of primary amino groups | Hemoglobin |
| Example 5 | DAMDPA | 2 | 3.34 |
| Comparative Example 10 | - | - | 0.73 |

From Figs. 15 and 16 and Table 2, it is found that in the sample analysis method of Examples in which the separation of hemoglobin was performed using an alkaline solution containing a cationic polymer and a cationic low-molecular compound having two or more primary amino groups, as compared with the sample analysis method of Comparative Examples in which the separation of hemoglobin was performed using an alkaline solution not containing a cationic low-molecular compound having two or more primary amino groups, the maximum absorbance variation of hemoglobin was large, the peak in the drawing was not broad, and thus the separation accuracy was excellent.

### <<Example 6>>

A sample containing transferrin was prepared, and diluted (dilution rate 7 times (volume basis)) with the diluent 1 (pH 8.8) to obtain Sample D.

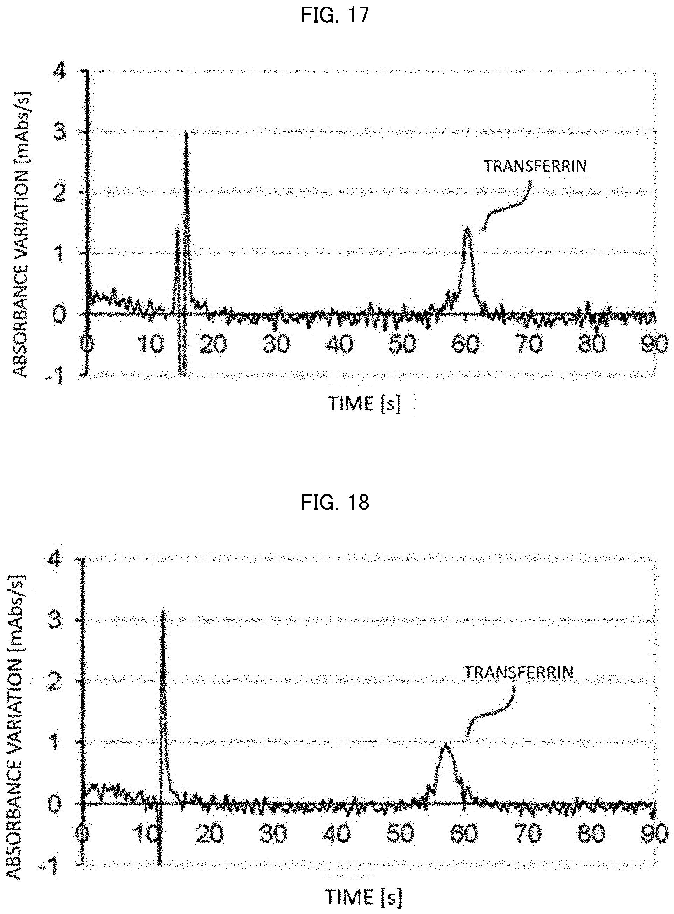

An electropherogram was obtained in the same manner as in Example 1, except that Sample A was changed to Sample D. The obtained electropherogram is shown in Fig. 17.

In Table 3, the maximum absorbance variation for transferrin was summarized.

### <<Comparative Example 11>>

An electropherogram was obtained in the same manner as in Comparative Example 1, except that Sample A was changed to Sample D. The obtained electropherogram is shown in Fig. 18.

In Table 3, the maximum absorbance variation for transferrin was summarized.

**[Table 3]**

| | Low-molecular compound | | Maximum absorbance variation (mAbs/s) |
|---|---|---|---|
| | Type | Number of primary amino groups | Transferrin |
| Example 6 | DAMDPA | 2 | 1.42 |
| Comparative Example 11 | - | - | 0.98 |

From Figs. 17 and 18 and Table 3, it is found that in the sample analysis method of Examples in which the separation of transferrin was performed using an alkaline solution containing a cationic polymer and a cationic low-molecular compound having two or more primary amino groups, as compared with the sample analysis method of Comparative Examples in which the separation of transferrin was performed using an alkaline solution not containing a cationic low-molecular compound having two or more primary amino groups, the maximum absorbance variation of transferrin was large, the peak in the drawing was not broad, and thus the separation accuracy was excellent.

### <<Example 7>>

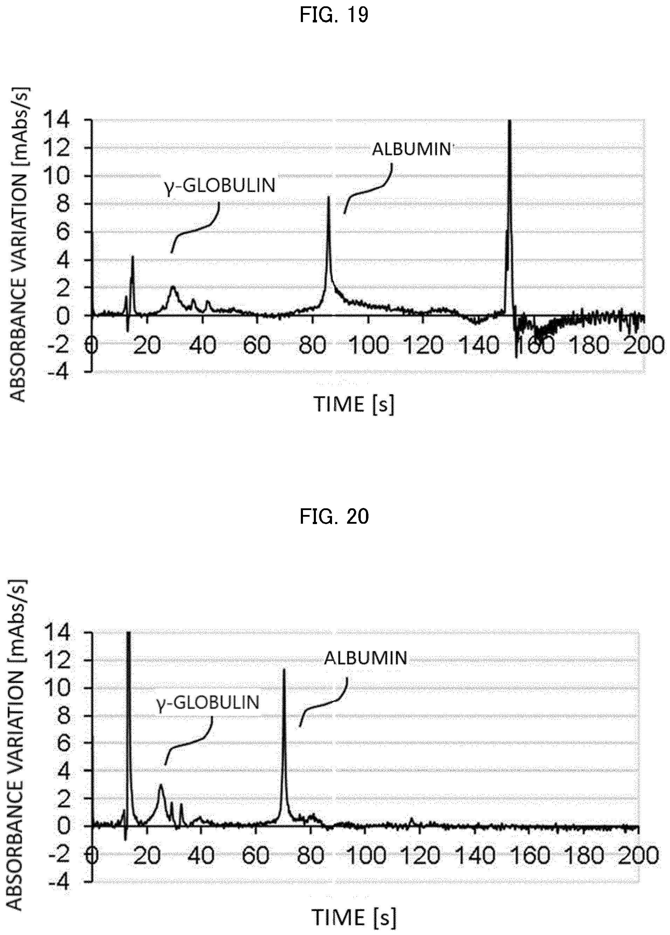

An electropherogram was obtained in the same manner as in Example 1, except that human biologically derived serum samples obtained from different donors were used. The obtained electropherogram is shown in Fig. 19.

In Table 4, the maximum absorbance variation for each of albumin and γ-globulin was summarized.

### <<Example 8>>

An electropherogram was obtained in the same manner as in Example 7, except that the diluent 1 was changed to the following diluent 2. The obtained electropherogram is shown in Fig. 20.

In Table 4, the maximum absorbance variation for each of albumin and γ-globulin was summarized.

In the diluent 2, hydrochloric acid and water were added until the pH of the solution reached 8.8.

### (Diluent 2)

· Polyethyleneimine (cationic polymer, manufactured by Wako, weight average molecular weight: 70,000) 0.75 mass%
· Non-surfactant-type sulfobetaine (NDSB-201 manufactured by Tokyo Chemical Industry Co., Ltd., 3-(1-pyridino)propanesulfonic acid) 8.06 mass%
· Sucrose 0.787 mass%
· Sodium chloride 0.26 mass%
· Sodium azide 0.018 mass%
· EMULGEN LS-110 (manufactured by Kao Corporation) 0.4 mass%
· Hydrochloric acid
· Water

### <<Comparative Example 12>>

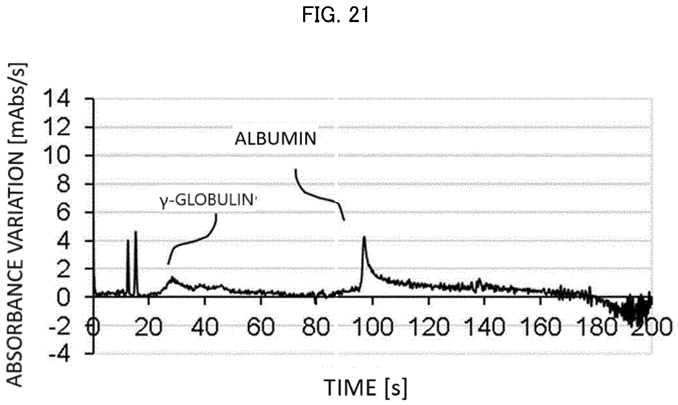

An electropherogram was obtained in the same manner as in Example 7, except that the specific alkaline solution 1 was changed to an alkaline solution 10. The obtained electropherogram is shown in Fig. 21.

In Table 4, the maximum absorbance variation for each of albumin and γ-globulin was summarized.

**[Table 4]**

| | Low-molecular compound | | Maximum absorbance variation (mAbs/s) | |
|---|---|---|---|---|
| | Type | Number of primary amino groups | Albumin | γ-Globulin |
| Example 7 | DAMDPA | 2 | 8.49 | 2.11 |
| Example 8 | DAMDPA | 2 | 11.34 | 3.01 |
| Comparative Example 12 | - | - | 4.26 | 1.44 |

From Figs. 19 to 21 and Table 4, it is found that in the sample analysis method of Examples in which the separation of albumin and γ-globulin was performed using an alkaline solution containing a cationic polymer and a cationic low-molecular compound having two or more primary amino groups, as compared with the sample analysis method of Comparative Examples in which the separation of albumin and γ-globulin was performed using an alkaline solution not containing a cationic low-molecular compound having two or more primary amino groups, the maximum absorbance variation of each of albumin and γ-globulin was large, the peak in the drawing was not broad, and thus the separation accuracy was excellent.

From Figs. 19 and 20 and Table 4, it is found that in the sample analysis method of Example 8 in which the separation of albumin and γ-globulin was performed using a sample containing a non-surfactant-type sulfobetaine, as compared with the sample analysis method of Example 7 in which the separation of albumin and γ-globulin was performed using a sample not containing a non-surfactant-type sulfobetaine, the maximum absorbance variation of each of albumin and γ-globulin was large, the peak in the drawing was not broad, and thus the separation accuracy was excellent. It is also found that the separation time was shortened.

## Claims

1. A sample analysis method, comprising:
a separation step of separating a protein from a sample comprising the protein, in an alkaline solution by capillary electrophoresis,
wherein separation of the protein is performed in a presence of a cationic compound having two or more primary amino groups, and
the alkaline solution comprises a cationic polymer,
and wherein the alkaline solution comprises the cationic compound, and
**characterized in that**
the cationic compound comprises at least one selected from the group consisting of 3,3'-diamino-N-methyldipropylamine, N,N'-bis(3-aminopropyl)ethylenediamine, 3,3'-diaminodipropylamine, tris(2-aminoethyl)amine, 1,2-diaminopropane, ethylenediamine, 1,3-diaminopentane, amidol, and 2,2'-oxybis(ethylamine).

2. The sample analysis method according to claim 1, wherein a content ratio of the cationic compound with respect to a total mass of the alkaline solution is from 0.01 mass% to 10 mass%.

3. The sample analysis method according to claim 1 or 2, wherein a ratio of a content ratio of the cationic compound to a content ratio of the cationic polymer in the alkaline solution is from 0.001 to 1000.

4. The sample analysis method according to claim 1, wherein the protein comprises at least one selected from the group consisting of hemoglobin, albumin, γ-globulin, and transferrin.

5. The sample analysis method according to claim 1, wherein a weight average molecular weight of the cationic polymer is from 15,000 to 150,000.

6. The sample analysis method according to claim 1, wherein a content ratio of the cationic polymer with respect to a total mass of the alkaline solution is from 0.01 mass% to 10 mass%.

7. The sample analysis method according to claim 1, wherein a pH of the alkaline solution is from 8.5 to 12.0.

8. A capillary electrophoresis solution, comprising:
a cationic polymer; and
a cationic compound having two or more primary amino groups,
wherein the capillary electrophoresis solution is used for separation of a protein by capillary electrophoresis,
and **characterized in that** the cationic compound comprises at least one selected from the group consisting of 3,3'-diamino-N-methyldipropylamine, N,N'-bis(3-aminopropyl)ethylenediamine, 3,3'-diaminodipropylamine, tris(2-aminoethyl)amine, 1,2-diaminopropane, ethylenediamine, 1,3-diaminopentane, amidol, and 2,2'-oxybis(ethylamine).

9. A sample analysis kit, comprising:
a container comprising the capillary electrophoresis solution according to claim 8; and
an electrophoresis chip comprising a sample holding tank, an electrophoretic liquid holding tank, and a capillary flow path,
wherein the sample holding tank and the electrophoretic liquid holding tank are communicated with each other via the capillary flow path.

## Patentansprüche

1. Probenanalyseverfahren, umfassend:
einen Trennschritt zum Trennen eines Proteins aus einer Probe, umfassend das Protein, in einer alkalischen Lösung mittels Kapillarelektrophorese,
wobei eine Trennung des Proteins in Gegenwart einer kationischen Verbindung mit zwei oder mehr primären Aminogruppen durchgeführt wird, und
die alkalische Lösung ein kationisches Polymer umfasst,
und wobei die alkalische Lösung die kationische Verbindung umfasst, und **dadurch gekennzeichnet, dass**
die kationische Verbindung mindestens eines umfasst, ausgewählt aus der Gruppe bestehend aus 3,3'-Diamino-N-methyldipropylamin, N,N'-Bis(3-aminopropyl)ethylendiamin, 3,3'-Diaminodipropylamin, Tris(2-aminoethyl)amin, 1,2-Diaminopropan, Ethylendiamin, 1,3-Diaminopentan, Amidol und 2,2'-Oxybis(ethylamin).

2. Probenanalyseverfahren nach Anspruch 1, wobei ein Gehaltsverhältnis der kationischen Verbindung bezogen auf eine Gesamtmasse der alkalischen Lösung zwischen 0,01 Gew.-% und 10 Gew.-% liegt.

3. Probenanalyseverfahren nach Anspruch 1 oder 2, wobei ein Verhältnis eines Gehaltsverhältnisses der kationischen Verbindung zum Gehaltsverhältnis des kationischen Polymers in der alkalischen Lösung zwischen 0,001 und 1000 liegt.

4. Probenanalyseverfahren nach Anspruch 1, wobei das Protein mindestens eines umfasst, ausgewählt aus der Gruppe bestehend aus Hämoglobin, Albumin, γ-Globulin und Transferrin.

5. Probenanalyseverfahren nach Anspruch 1, wobei ein gewichtsmittleres Molekulargewicht des kationischen Polymers zwischen 15.000 und 150.000 liegt.

6. Probenanalyseverfahren nach Anspruch 1, wobei ein Gehaltsverhältnis des kationischen Polymers bezogen auf eine Gesamtmasse der alkalischen Lösung zwischen 0,01 Gew.-% und 10 Gew.-% liegt.

7. Probenanalyseverfahren nach Anspruch 1, wobei ein pH-Wert der alkalischen Lösung zwischen 8,5 und 12,0 liegt.

8. Kapillarelektrophorese-Lösung, umfassend:
ein kationisches Polymer; und
eine kationische Verbindung mit zwei oder mehr primären Aminogruppen,
wobei die Kapillarelektrophorese-Lösung zur Trennung eines Proteins mittels Kapillarelektrophorese verwendet wird,
und **dadurch gekennzeichnet, dass**
die kationische Verbindung mindestens eine Verbindung umfasst, die ausgewählt ist aus der Gruppe bestehend aus 3,3'-Diamino-N-methyldipropylamin, N,N'-Bis(3-aminopropyl)ethylendiamin, 3,3'-Diaminodipropylamin, Tris(2-aminoethyl)amin, 1,2-Diaminopropan, Ethylendiamin, 1,3-Diaminopentan, Amidol und 2,2'-Oxybis(ethylamin).

9. Probenanalyse-Kit, umfassend:
einen Behälter, der die Kapillarelektrophorese-Lösung nach Anspruch 8 umfasst; und
einen Elektrophorese-Chip, der einen Probenbehälter, einen Behälter für Elektrophoreseflüssigkeit und einen Kapillarflussweg umfasst,
wobei der Probenbehälter und der Behälter für Elektrophoreseflüssigkeit über den Kapillarflussweg miteinander verbunden sind.

## Revendications

1. Procédé d'analyse d'échantillons, comprenant :
une étape de séparation consistant à séparer une protéine d'un échantillon comprenant la protéine, dans une solution alcaline par électrophorèse capillaire,
dans lequel la séparation de la protéine est effectuée en présence d'un composé cationique présentant deux groupes amino primaires ou plus, et
la solution alcaline comprend un polymère cationique,
et dans lequel la solution alcaline comprend le composé cationique et **caractérisé en ce que**
le composé cationique comprend au moins l'un sélectionné à partir du groupe constitué de la 3,3'-diamino-N-méthyldipropylamine, la N,N'-bis(3-aminopropyl)éthylènediamine, la 3,3'-diaminodipropylamine, la tris(2-aminoéthyl)amine, du 1,2-diaminopropane, l'éthylènediamine, du 1,3-diaminopentane, l'amidol et la 2,2'-oxybis(éthylamine).

2. Procédé d'analyse d'échantillons selon la revendication 1, dans lequel un rapport de contenu du composé cationique par rapport à une masse totale de la solution alcaline est de 0,01 % en masse à 10 % en masse.

3. Procédé d'analyse d'échantillons selon la revendication 1 ou la revendication 2, dans lequel un rapport d'un rapport de contenu du composé cationique sur un rapport de contenu du polymère cationique dans la solution alcaline est de 0,001 à 1 000.

4. Procédé d'analyse d'échantillons selon la revendication 1, dans lequel la protéine comprend au moins l'une sélectionnée à partir du groupe constitué de l'hémoglobine, de l'albumine, de la γ-globuline et de la transferrine.

5. Procédé d'analyse d'échantillons selon la revendication 1, dans lequel un poids moléculaire moyen en poids du polymère cationique est de 15 000 à 150 000.

6. Procédé d'analyse d'échantillons selon la revendication 1, dans lequel un rapport de contenu du polymère cationique par rapport à une masse totale de la solution alcaline est de 0,01 % en masse à 10 % en masse.

7. Procédé d'analyse d'échantillons selon la revendication 1, dans lequel un pH de la solution alcaline est de 8,5 à 12,0.

8. Solution d'électrophorèse capillaire, comprenant :
un polymère cationique ; et
un composé cationique présentant deux groupes amino primaires ou plus,
dans laquelle la solution d'électrophorèse capillaire est utilisée pour la séparation d'une protéine par électrophorèse capillaire,
et **caractérisée en ce que**
le composé cationique comprend au moins l'un sélectionné à partir du groupe constitué de la 3,3'-diamino-N-méthyldipropylamine, la N,N'-bis(3-aminopropyl)éthylènediamine, la 3,3'-diaminodipropylamine, la tris(2-aminoéthyl)amine, du 1,2-diaminopropane, l'éthylènediamine, du 1,3-diaminopentane, l'amidol et la 2,2'-oxybis(éthylamine).

9. Kit d'analyse d'échantillons, comprenant :
un récipient comprenant la solution d'électrophorèse capillaire selon la revendication 8 ; et
une puce d'électrophorèse comprenant un réservoir de stockage d'échantillons, un réservoir de stockage de liquide électrophorétique et un chemin d'écoulement capillaire,
dans lequel le réservoir de stockage d'échantillons et le réservoir de stockage de liquide électrophorétique sont mis en communication l'un avec l'autre par l'intermédiaire du chemin d'écoulement capillaire.
